# EUROPÄISCHE PATENTSCHRIFT

(11) **EP 1 870 021 B1**
(45) Veröffentlichungstag und Bekanntmachung des Hinweises auf die Patenterteilung: **17.05.2017**
(21) Anmeldenummer: 06012842.8
(22) Anmeldetag: 22.06.2006
(51) Int. Cl.: A61B 1/06, A61B 1/24, A61C 1/08

(54) **Medizinischer Handgriff mit Beleuchtungsvorrichtung**
Medical handpiece with illumination device
Pièce à main médicale avec dispositif d'illumination

(43) Veröffentlichungstag der Anmeldung: 26.12.2007
(73) Patentinhaber: W & H Dentalwerk Bürmoos GmbH, 5111 Bürmoos (AT)
(72) Erfinder: Brandstätter, Andreas, 5120 St. Pantaleon 208 (AT); Schalda, Anton, 5121 Ostermiething (AT); Wendtner, Wolfgang, 5112 Lamprechtshausen (AT)

(56) Entgegenhaltungen:
- EP-A2- 1 093 765
- DE-A1- 10 359 501
- DE-U1-202005 002 341
- GB-A- 2 395 566
- US-A- 3 109 238
- US-A- 6 161 937
- US-A1- 2006 077 671

## Beschreibung

Die vorliegende Erfindung betrifft einen medizinischen Handgriff mit einer Beleuchtungsvorrichtung mit zumindest einem optischen Halbleiterbauelement als Lichtquelle und ein Verfahren zur Herstellung eines derartigen Handgriffs.

Ein medizinischer Handgriff mit einer Beleuchtungsvorrichtung mit einer Leuchtdiode. (LED) ist aus der EP 1.093.765 A2 bekannt. Die Beleuchtungsvorrichtung besteht aus einem Halter mit einem U-förmigen Querschnitt in dem die LEDs aufgenommen und mit einem transparenten, wärmebeständigen Kunstharz vergossen und damit von der Umgebung abgedichtet sind. Die Lichtabgabe erfolgt durch das Kunstharz. Durch diesen Aufbau ist die Beleuchtungsvorrichtung in einem Autoklaven sterilisierbar.

Die Beleuchtungsvorrichtung dieses medizinischen Handgriffs weist jedoch mehrere Nachteile auf. Primär vergilben bekannte und für diesen Einsatzzweck geeignete Kunstharze nach mehrmaliger Sterilisation, so dass die Lichttransmission und die Lichtausbeute während der Lebensdauer des Handgriffs merkbar schlechter wird. Ein weiterer Nachteil besteht darin, dass die Kunstharze vergleichsweise weiche Oberflächen haben. Da das Licht über das Kunstharz abgegeben wird und das Kunstharz somit an der Oberfläche der Beleuchtungsvorrichtung angeordnet ist, besteht die Gefahr der mechanischen Beschädigung des Kunstharzmantels. Dadurch wird die Lichtabgabe der Beleuchtungsvorrichtung weiter verschlechtert, z.B. durch Kratzer, an denen das Licht gebrochen oder abgelenkt wird. Des Weiteren besteht die Gefahr, dass bei tiefer gehenden mechanischen Schäden Risse entstehen, die bis zu den LEDs reichen, so dass diese insbesondere bei der Sterilisation nicht mehr vor Wasserdampf oder Chemikalien geschützt sind. Auch kann die zur Behandlungsstelle weisende Vorderfläche des Harzes im Wesentlichen nur plan ausgebildet werden, so dass eine weitere Aufbereitung des abgestrahlten Lichts nicht möglich ist.

Aus dem Stand der Technik sind des Weiteren gekapselte LEDs bekannt, deren Umhüllung aus zumindest einem, oft mehreren metallischen Bauteilen bestehen, siehe zum Beispiel US 4,295,152. Diese gekapselten LEDs können in medizinischen Handstücken nicht eingesetzt werden, da sie den Vorgaben zur elektrischen Sicherheit nicht genügen. Sind die Handgriffe mit Stromquellen verbindbar, zum Beispiel wenn sie eine Beleuchtungsvorrichtung aufweisen oder mit einem Elektromotor betrieben werden, so muss sicher gestellt sein, dass weder auf den Anwender noch auf den Patienten während des Betriebs oder bei Auftreten eines Defekts Strom übertragen wird. Werden herkömmliche gekapselte LEDs mit metallischen Bauteilen verwendet, so sind zur Einhaltung dieser Vorgaben definierte Abstände zwischen der elektrisch leitenden, metallischen Außenhülse des Handgriffs und spannungsführenden Bauteilen, zum Beispiel der gekapselten LED, vorgeschrieben. Diese definierten Abstände sind von verschiedenen Faktoren abhängig und betragen zum Beispiel bei einer pro LED notwendigen Versorgungsspannung von 2,8 - 3,5 V zumindest 0,4 mm (Luftstrecke) bzw. 0,8 mm (Kriechstrecke), bei Wasser führenden Handgriffen, zum Beispiel für die Abgabe eines Wassersprays, 1,6 mm (Luftstrecke) bzw. 3,4 mm (Kriechstrecke). Da der Durchmesser der Handgriffe mit etwa 1 - 2 cm vergleichsweise gering ist, ist es häufig nicht möglich, diese Vorgaben einzuhalten, so dass die bekannten mit Metallhülsen gekapselten LEDs für medizinische Handgriffe nicht verwendbar sind.

Aus dem Dokument DE 103 59 501 A1 ist ein dentales Funktionshandstück mit einer am Vorderende angeordneten Beleuchtungsvorrichtung mit LEDs, die In einem Grundkörper aufgenommen sind, bekannt.

Die Patentschrift US 3.109.238 A offenbart einen dentalen Handgriff mit einer mehrere Glühbirnen umfassenden Beleuchtungsvorrichtung, wobei die Glühbirnen teilweise in einem als Lichtleiter dienenden Grundkörper aufgenommen sind.

Die Patentarimeldung US 2006/0077671 A1 offenbart eine längliche, rohrförmige Beleuchtungsvorrichtung mit mehreren LEDs, die lösbar an der Außenseite eines medizinischen Instruments befestigbar ist.

Der vorliegenden Erfindung liegt daher die Aufgabe zugrunde einen medizinischen Handgriff mit einer alternativen Beleuchtungsvorrichtung zu schaffen. Der Handgriff soll insbesondere eine Beleuchtungsvorrichtung aufweisen, die während der gesamten Lebensdauer des Handgriffs eine verbesserte, möglichst gleich bleibende Lichtabgabe gewährleistet, deren Oberfläche insbesondere mechanischen Beschädigungen besser widerstehen kann, die sterilisierbar ist, die eine Aufbereitung des abgestrahlten Lichts zulässt und die die Vorgaben zur elektrischen Sicherheit erfüllt.

Diese Aufgabe wird gemäß der vorliegenden Erfindung durch einen Handgriff mit den Merkmalen des Anspruchs 1 und ein Verfahren zur Herstellung eines derartigen Handgriffs mit den Merkmalen des Anspruchs 15 gelöst. Besonders vorteilhafte Ausführungsformen sind in den Unteransprüchen angeführt.

Der Aufbau der Beleuchtungsvorrichtung des Handgriffs mit einem Grundkörper und zumindest einer in der Wand des Grundkörpers angeordneten Sacklochbohrung und einem Lichtabgabefenster, das durch jenen Teil der Wand, der sich vom Boden der Sacklochbohrung bis zum ersten Ende des Grundkörpers erstreckt, gebildet ist, hat den Vorteil, dass damit eine der Behandlungsstelle zugewandte Oberfläche geschaffen wird, die mechanischen Beschädigungen besser widerstehen kann. Des Weiteren bietet die Oberfläche eine einfache Möglichkeit, das vom optischen Halbleiterbauelement abgestrahlte Licht aufzubereiten, in dem die Oberfläche des Grundkörpers als plane, polierte Oberfläche oder als optisches Element ausgebildet ist, zum Beispiel als konvexe oder konkave Linse. Ein bevorzugt einstückig mit dem Grundkörper ausgebildetes optisches Element hat den zusätzlichen Vorteil, dass es nicht auf der Beleuchtungsvorrichtung befestigt werden muss und damit auch keine Gefahr besteht, dass es sich zum Beispiel aufgrund der bei der Sterilisation herrschenden Umgebungsbedingungen von der Beleuchtungsvorrichtung abhebt und in einem damit entstehenden Zwischenraum Keimen die Möglichkeit zur Ansiedlung bietet oder dass es sich vollkommen davon ablöst. An die Oberfläche kann auch in einfacher Weise ein Lichtleiter angeschlossen werden.

Die zumindest eine Sacklochbohrung kann verschiedene Formen aufweisen, sie kann zum Beispiel ein im wesentlichen zylindrischer Hohlraum sein, der nur unwesentlich größer als ein darin aufgenommenes optisches Halbleiterbauelement ist, wobei in die Wand des Grundkörpers auch mehrere dieser Hohlräume gleichmäßig oder unregelmäßig verteilt sein können. Alternativ kann die Sacklochbohrung größer, zum Beispiel kreisbogenförmig ausgebildet sein und sich um die Längsachse des Grundkörpers erstrecken, wobei wiederum auch mehrere, zum Beispiel zwei viertelkreisbogenförmige Sacklochbohrungen vorhanden sein können. Schließlich kann die Sacklochbohrung auch als ein durchgehender, rund um die Längsachse des Grundkörpers verlaufender Ring ausgebildet sein. Ist die Sacklochbohrung derart ausgebildet, dass sie mehrere optische Halbleiterbauelemente aufnehmen kann, so ist damit eine besonders hohe Lichtintensität auf einer kleinen Fläche erzielbar.

Der Grundkörper besteht aus elektrisch nicht leitendem und wiederholtes Sterilisieren widerstehendem Material. Des Weiteren muss das Lichtabgabefenster für die von dem zumindest einen optischen Halbleiterbauelement abgestrahlte Wellenlänge oder für ein Spektrum, das von einem in der Beleuchtungsvorrichtung angeordneten Farbkonversionsmaterial erzeugt wird, transparent sein. Als Materialien für den Grundkörper und das Lichtabgabefenster sind bevorzugt Kunststoffe, insbesondere Polyphenylsulfone, Polyethersulfone, Polycarbonate oder Polyamide oder Glas verwendbar. Diese Materialien sind auch elektrisch isolierend, so dass die elektrische Sicherheit gewährleistet ist. Bevorzugt werden als Material für den Grundkörper teilkristalline Polyamide eingesetzt, die zum Beispiel in unterschiedlichen Ausführungsformen als Formmasse unter der Bezeichnung Trogamid^{®} von der Firma Degussa AG, Deutschland angeboten werden. Diese teilkristallinen Polyamide sind auch besonders kratzfest und abriebbeständig, so dass sie mechanischen Belastungen gut widerstehen.

Unter dem Begriff "wiederholtes Sterilisieren widerstehendes Material" bzw. "sterilisierbares Material" wird jedes Material verstanden, das auch nach mehrmaligem Einbringen in eine aggressive Umgebung mit einer Temperatur von zumindest 120°C, bevorzugt von 130°C, besonders bevorzugt von 134°C und mit Wasserdampf und / oder in eine Umgebung mit Chemikalien, insbesondere Desinfektionsmittel keine wesentlichen Veränderungen oder Schäden aufweist, so dass ein durch ein derartiges Material umschlossener und von der Umwelt hermetisch abgeschlossener Innenraum auch nach mehrmaligem Einbringen des Materials in diese aggressive Umgehung hermetisch verschlossen bleibt. Im Innenraum aufgenommene Bauteile, insbesondere optische Halbleiterbauelemente sind somit durch eine Kapselung mit einem derartigen Material sowohl während des Sterilisationsvorganges als auch danach vor Wasserdampf, Chemikalien, Partikeln, Schmiermitteln oder anderen Verschmutzungen geschützt und können so ihre Funktionsfähigkeit aufrechterhalten.

Die mit dem Grundkörper verbindbare Abdeckvorrichtung kann in einem ersten Ausführungsbeispiel durch zumindest ein Vergussmaterial, insbesondere Kunstharze wie Epoxidharz, Silikonharze oder Polyamidharze gebildet sein. Diese Ausführungsform ist in ihrer Herstellung sehr einfach und kostengünstig. Sie ermöglicht auch mehrere, gegebenenfalls unterschiedliche optische Halbleiterbauelemente In jeweils einer eigenen Sacklochbohrung anzuordnen und hermetisch zu verschließen, wobei besonders bevorzugt die Anordnung der optischen Halblelterbauelemente unregelmäßig ist, so dass spezielle Lichtverteilungsmuster realisierbar sind. Bei dieser Ausführungsform kann zumindest ein Teil der Vergussmasse in der Sacklochbohrung aufgenommen sein.

In einem zweiten Ausführungsbeispiel umfasst die Abdeckvorrichtung zumindest einen Verschlusskörper, der bevorzugt zylindrisch, stab- oder plattenförmig geformt ist Die Abdeckvorrichtung besteht bevorzugt aus Kunststoff, insbesondere aus Polyphenylsulfon, Polyethersulfon, Polycarbonat oder Polyamid, Glas oder Keramik. Diese Materialien sind, wie auch die oben angeführten Vergussmaterialien elektrisch isoilerend, so dass die elektrische Sicherheit gewährleistet ist. Bevorzugt werden als Material für den Grundkörper teilkristalline Polyamide eingesetzt, die zum Beispiel in unterschiedlichen Ausführungsformen als Formmasse unter der Bezeichnung Trogamid^{®} von der Firma Degussa AG, Deutschland angeboten werden. Der Vorteil dieses zweiten Ausführungsbeispiels liegt vor allem in der sehr zuverlässigen Verbindung, die zwischen der Abdeckvorrichtung und dem Grundkörper herstellbar ist, insbesondere durch Laserverschweißen, so dass eine äußerst dauerhafte Abdichtung des Innenraums, in dem das zumindest eine optischen Halbleiterbauelement aufgenommen ist, herstellbar ist Wie weiter unten noch detailliert beschrieben ist auch die Abdichtung von gegebenenfalls vorhandenen elektrischen Kontakten zur Stromversorgung des optischen Halbleiterbauelements bei Vorhandensein eines Verschlusskörpers sehr zuverlässig realisierbar.

Es Ist nicht zwingend notwendig, dass der Verschlusskörper der Abdeckvorrichtung und der Grundkörper aus denselben Materialien bestehen. Bevorzugt sollen die unterschiedlichen Materialien jedoch einen ähnlichen oder annährend gleichen Wärmeausdehnungskoeffizienten haben, so dass insbesondere bei Erwärmung der Beleuchtungsvorrichtung während der Sterilisation keine Risse oder undichte Stellen zwischen den beiden Bauteilen entstehen, durch die Wasserdampf, Chemikallen oder andere Verschmutzungen eindringen können. Besonders bevorzugt bestehen der Verschlusskörper der Abdeckvorrichtung und der Grundkörper jedoch aus denselben Materialien um eine möglichst hohe Dichtwirkung zu erreichen.

Die Verbindung des Grundkörpers mit der Abdeckvorrichtung kann durch Verschweißen, zum Beispiel Ultraschall- oder Laserverschweißen, Verpressen, Verkleben oder mittels Dichtelementen, zum Beispiel O-Ringen oder Gummiringen erfolgen. Bevorzugt wird das Laser-Durchstrahl-Schweißverfahren verwendet, bei dem eines der beiden zu verbindenden Bauteile für die Laserstrahlung transparent ist und das andere Bauteil die Laserstrahlung absorbiert. Durch die absorbierte Strahlung schmlizt dieses Bauteil oberflächlich auf und überträgt einen Teil der Wärme auf das transparente Bauteil, das ebenfalls oberflächlich aufschmilzt, so dass sich im Bereich der einander kontaktierenden Oberflächen die beiden Bauteile beim anschließenden Abkühlen verbinden. Vorteilhafterweise sind für die Anwendung dieses Verfahrens beide Bauteile aus dem gleichen Material hergestellt.

Bestehen der Grundkörper und die Abdeckvorrichtung aus Kunststoff, so können sie durch spanabhebende Bearbeitung aus einem Werkstück oder bevorzugt durch Spritzgießen hergestellt werden.

Das als Leuchtdiode (LED) ausgebildete zumindest eine optische Halbleiterbauelement kann auf einem Träger, zum Beispiel einer Platine oder einer Multilayer-Keramik angeordnet sein. Die zumindest eine LED kann jedoch auch ohne Träger direkt in die Sacklochbohrung eingesetzt sein und darin vergossen oder durch das Abdeckteil hermetisch verschlossen sein. Diese Ausführungsform hat den Vorteil einer besonders geringen Bauhöhe und damit eines besonders geringen Platzbedarfs im oder am Handgriff.

Die elektrische Versorgungseinrichtung umfasst in einem ersten Ausführungsbeispiel zumindest einen ersten und zweiten elektrischen Kontakt zur Stromversorgung des zumindest einen optischen Halbleiterbauelements. Die Kontakte können zum Beispiel aus Kontaktstiften oder aus Litzen gebildet sein. Sind mehrere Halbleiterbauelemente vorhanden, so können diese in Serie oder parallel angeordnet sein, so dass entsprechend mehrere elektrische Kontakte vorhanden sind. Die Verbindung zwischen der zumindest einen LED und den elektrischen Kontakten kann durch Bondingdrähte oder über Platinenleitungen erfolgen.

Ist die Abdeckvorrichtung durch zumindest ein Vergussmaterial gebildet, so werden bevorzugt die elektrischen Kontakte zuerst mit der zumindest einen LED verbunden, in die Sacklochbohrung zumindest teilweise eingebracht und anschließend vergossen. Alternativ ist es auch möglich, die Kontakte nicht durch das Vergussmaterial zu führen, sondern durch Bohrungen im Grundkörper, wobei die Abdichtung in gleicher Weise erfolgt wie dies im Folgenden für einen Verschlusskörper einer Abdeckvorrichtung beschrieben ist.

Umfasst die Abdeckvorrichtung zumindest einen Verschlusskörper, so sind die zumindest zwei elektrischen Kontakte durch Bohrungen entweder im Verschluss- oder im Grundkörper geführt, wobei die Bohrungen bis in die Sacklochbohrung / den hermetisch abgedichteten Innenraum reichen. Die Abdichtung der elektrischen Kontakte kann wiederum durch Verschweißen, zum Beispiel Ultraschall- oder Laserverschweißen, Verpressen, Verkleben oder mittels Dichtelementen erfolgen. Bevorzugt wird der Verschluss- oder Grundkörper jedoch durch Spritzgießen hergestellt, wobei die elektrischen Kontakte vor Beginn der Spritzgießherstellung in die Spritzgießform eingebracht und so mitverspritzt werden, wodurch eine äußerst zuverlässige Abdichtung erreicht wird.

In einem zweiten Ausführungsbeispiel ist eine induktive elektrische Versorgungseinrichtung zur Stromversorgung des zumindest einen optischen Halbleiterbauelements vorgesehen, mit zumindest einer ersten Spule, die im hermetisch abgedichteten Innenraum der Beleuchtungsvorrichtung angeordnet ist, und zumindest einer zweiten Spule, die außerhalb des hermetisch abgedichteten Innenraums angeordnet ist. Bevorzugt sind zwischen der ersten Spule und dem zumindest einen optischen Halbleiterbauelement weitere elektronische Bauelemente, wie zum Beispiel Gleichrichter, Stromregler, AC-DC-Wandler, Kondensatoren oder Widerstände vorgesehen, um das zumindest eine optische Halbleiterbauelement mit der benötigten Gleichspannung zu versorgen bzw. eine konstante Stromversorgung sicherzustellen. Ist aufgrund der beengten Platzverhältnisse eine Verwendung dieser elektronischen Bauteile nicht möglich, so kann die induktive Versorgungseinrichtung oberhalb der so genannten Flimmerfrequenz (entsprechend etwa 25 Hz Wechselspannung) betrieben werden, wodurch für das menschliche Auge der Eindruck einer konstanten, nicht unterbrochenen Lichtabgabe des zumindest einen optischen Halbleiterbauelements entsteht.

Als primäre Stromquelle, mit der die elektrischen Kontakte oder die induktive Versorgungseinrichtung verbunden oder verbindbar sind, kann eine Batterie, ein Akkumulator oder das Stromnetz dienen. Bevorzugt wird die von den genannten Stromquellen zur Verfügung gestellte Energie entsprechend den Anforderungen der Halbleiterbauelemente mittels elektronischer oder elektrischer Bauelemente aufbereitet.

In einem bevorzugten Ausführungsbeispiel ist die Beleuchtungsvorrichtung als Ringlicht ausgebildet, das bevorzugt sehr nahe am oder direkt um das Werkzeug angebracht ist. Damit ist kein Lichtleiter notwendig, der das Ucht durch den Handgriff an das werkzeugseitige Ende des Handgriffs leitet, wodurch die Strahlungsverluste deutlich reduziert werden. Bei der als Ringlicht ausgebildeten Beleuchtungsvorrichtung haben zumindest der Grundkörper, gegebenenfalls auch der Träger und die Abdeckvorrichtung eine Bohrung, in der das Werkzeug oder die Werkzeugaufnahme aufgenommen oder durchgeführt werden können.

Das Verfahren zur Herstellung eines medizinischen Handgriffs mit einer Beleuchtungsvorrichtung besteht aus den Schritten:
- Zur Verfügung stellen eines medizinischen Handgriffs,
- Zur Verfügung stellen einer Beleuchtungsvorrichtung mit zumindest einem optischen Halbleiterbauelement als Lichtquelle, wobei die Beleuchtungsvorrichtung des Weiteren umfasst:
   - einen Grundkörper mit einem ersten Ende und einem zweiten Ende,
   - zumindest eine in dem Grundkörper angeordnete Sacklochbohrung, die sich vom zweiten Ende des Grundkörpers In Richtung des ersten Endes erstreckt,
   - ein Uchtabgabefenster, das durch jenen Teil des Grundkörpers, der sich vom Boden der Sacklochbohrung bis zum ersten Ende des Grundkörpers erstreckt, gebildet ist,
   - eine Abdeckvorrichtung, die mit dem Grundkörper verbindbar ist und mit der Sacklochbohrung einen hermetisch abgedichteten Innenraum bildet,
   wobei das optische Halbleiterbauelement im hermetisch abgedichteten Innenraum angeordnet ist und über eine elektrische Versorgungseinrichtung mit einer Stromquelle verbunden oder verbindbar ist und wobei der Grundkörper und die Abdeckvorrichtung aus elektrisch nicht leitendem Material bestehen,
- Befestigen der Beleuchtungsvorrichtung in oder an dem medizinischen Handgriff.

Die Befestigung der Beleuchtungsvorrichtung kann an der Außenseite des medizinischen Handgriffs erfolgen, zum Beispiel durch eine Steck- oder Klemmverbindung. Bevorzugt ist die Beleuchtungsvorrichtung im Inneren des Handgriffs angeordnet, zum Beispiel durch eine Steck- oder Klemmverbindung oder durch Verkleben, wo sie für den Anwender weniger störend ist. Besonders bevorzugt ist die Beleuchtungsvorrichtung rund um das Werkzeug, zum Beispiel einen rotierenden Bohrer, eine hin- und herbewegbare Feile oder Säge oder ein dentales, in Schwingung versetzbares Zahnsteinentfemungswerkzeug angeordnet, so dass eine schattenfreie, lichtstarke Beleuchtung möglichst in der Nähe der Behandlungsstelle vorliegt. In diesem Fall kann die Beleuchtungsvorrichtung zumindest teilweise im Inneren des Handgriffs angeordnet sein, wobei die Oberfläche des Lichtabgabefensters gleichzeitig auch das Vorderende oder eine Seitenbegrenzung des Handgriffs bildet.

Die Erfindung wird nachfolgend anhand von bevorzugten Ausführungsbeispielen und Bezug nehmend auf die beigefügten Zeichnungen erläutert:
Figur 1 zeigt als Explosionsdarstellung ein erstes Ausführungsbeispiel der Beleuchtungsvorrichtung eines erfindungsgemäßen medizinischen Handgriffs.
Figur 2 zeigt einen Längsschnitt durch die zusammengesetzte Beleuchtungsvorrichtung der Figur 1.
Figur 3 zeigt ein zweites Ausführungsbeispiel der Beleuchtungsvorrichtung eines erfindungsgemäßen medizinischen Handgriffs.
Figur 4 zeigt einen erfindungsgemäßen medizinischen Handgriff in Form eines dentalen Zahnsteinentfernungsgeräts.

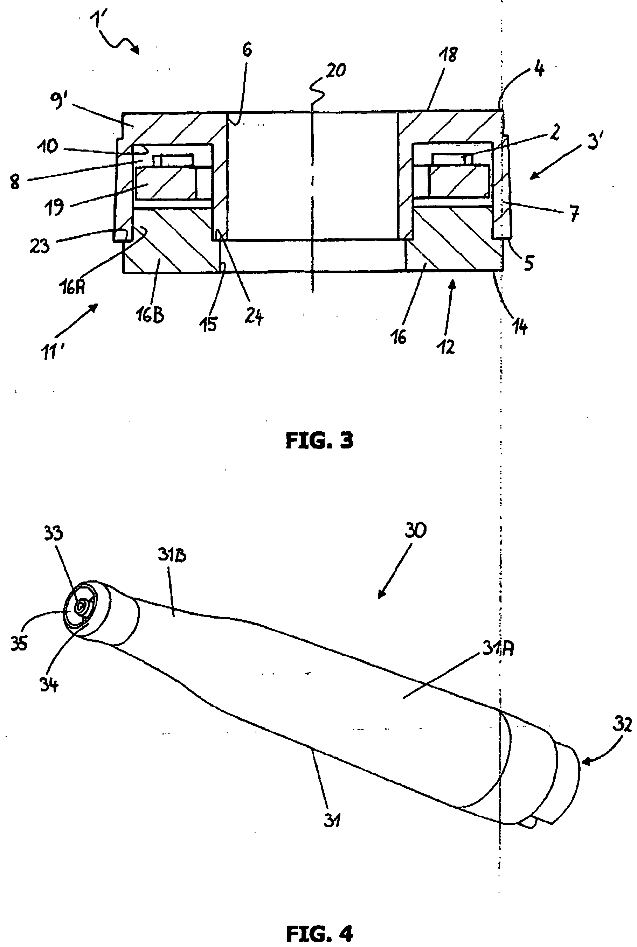

In Figur 4 ist beispielhaft für einen medizinischen Handgriff 30 ein Zahnsteinentfernungsgerät, oftmals auch als Scaler bezeichnet, dargestellt. Selbstverständlich ist der Begriff Handgriff jedoch nicht auf Scaler beschränkt sondern schließt auch alle anderen medizinisch, chirurgisch, orthopädisch, dental oder kosmetisch verwendbaren Handgriffe mit ein, insbesondere auch Handgriffe die gebogen sind oder gewinkelt zueinander angeordnete Abschnitte aufweisen, Handgriffe mit unterschiedlichen Antrieben oder Antriebsenergien, zum Beispiel mit Druckluft, Wasser oder Strom betriebene Handgriffe, Handgriffe mit unterschiedlichen Werkzeugen wie Bohrer, Sägen, Frasen, Bürsten, Prophy-Cups, Reamer, Shaver, medizinische Handgriffe zur Abgabe von Laserstrahlung oder Handgriffe, deren primäre Aufgabe die Abgabe von Licht zur Beleuchtung oder zur diagnostischen, therapeutischen oder prophylaktischen Behandlung ist. Unter dem Begriff Handgriff sind des Weiteren auch Teile solcher Handgriffe und Bauelemente wie Motore, Kupplungen, Adapter oder Zwischenstücke zur Verbindung von Bauteilen eines medizinischen Geräts zu verstehen.

Der Scaler umfasst eine längliche, zylindrische, Innen hohle Außenhülse 31, die in einen Griffteil 31A und einen Kopfteil 31B unterteilt Ist. Im Inneren der Außenhülse 31 können verschiedene Funktionstelle angeordnet sein, so zum Beispiel eine Antriebseinheit, Leitungen für Antriebs- und / oder Kühlmedien, Vibrationen dämpfende Einbauten, Regel- und Stelleinrichtungen. An dem dem Kopfteil 31B gegenüberliegenden Ende des Griffteils 31A ist eine Anschlussvorrlchtung 32, zum Anschluss des Handgriffs 30 an eine Energiequelle, zum Beispiel an eine Druckluft- oder Stromquelle, an eine oder mehrere Kühlmedienquellen und gegebenenfalls an eine Steuervorrichtung vorgesehen. Da die Anschlussvorrichtung 32 bzw. die verschiedenen Arten von Anschlussvorrichtungen, die mit einem derartigen Handgriff 30 verwendbar sind, bekannt sind, wird darauf nicht weiter eingegangen.

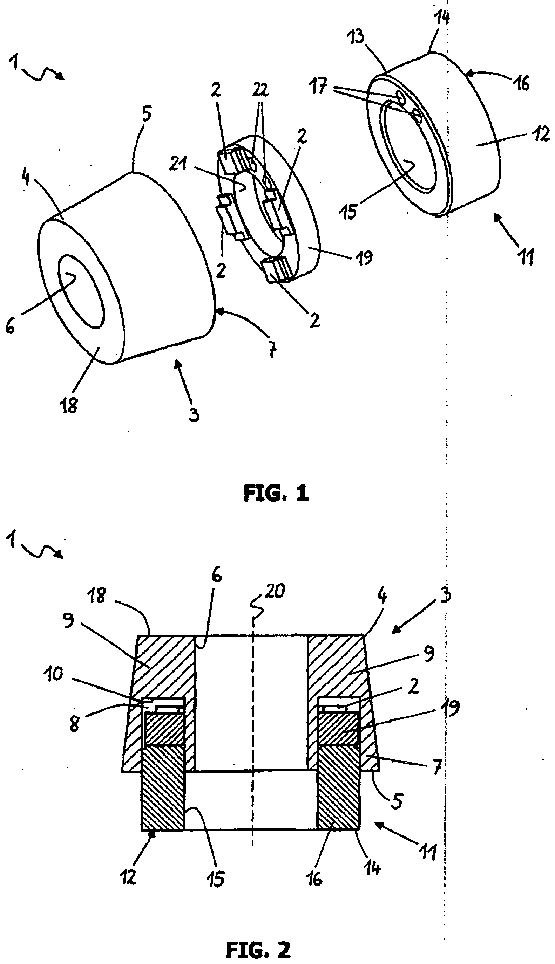

Am distalen Ende des Kopfteils 31B ist eine Werkzeugaufnahme 33 In Form eines hohlen Schaftes mit Innengewinde erkennbar, in die das Behandlungswerkzeug einschraubbar ist. Die Werkzeugaufnahme 33 ist vom hülsenförmig ausgebildeten, zylindrischen Vorderende 34 des Kopfteils 31 B umgeben, wobei das zylindrische Vorderende 34 von der Werkzeugaufnahme 33 beabstandet ist, wodurch ein Hohlraum 35 entsteht. In diesen Hohlraum 35 Ist eine als Ringlicht ausgebildete Beleuchtungsvorrichtung 1, wie sie zum Beispiel in den Figuren 1 - 3 dargestellt ist, einsetzbar. Die Beleuchtungsvorrichtung 1 ist somit im Wesentlichen im Handgriff 30 aufgenommen, wobei die Lichtabgabefläche 18 in etwa plan mit dem Vorderende 34 abschließt. Die Werkzeugaufnahme 33 ragt in oder durch eine oder mehrere Bohrungen 6, 15 der Beleuchtungsvorrichtung 1, so dass sie für den Anwender frei zugänglich ist und dieser das Behandlungswerkzeug problemlos In die Werkzeugaufnahme 33 einsetzen oder daraus lösen kann.

Beleuchtungsvorrichtung 1 ist gemäß den Figuren 1 - 3 dreiteilig aufgebaut und besteht aus einem Grundkörper 3, einer Abdeckvorrichtung 11 und einem Träger 19 in Form einer Platine. Der Grundkörper 3 hat eine schwach kegelförmige Außenform mit einem ersten Ende 4, das einen geringeren Durchmesser hat als das zweite Ende 5. Zwischen den beiden Enden 4, 5 verläuft eine konzentrisch zur Längsachse 20 der Beleuchtungsvorrichtung 1 angeordnete Bohrung 6, In die wie oben beschrieben die Werkzeugaufnahme 33 ragt, wenn die Beleuchtungsvorrichtung 1 am Handgriff 30 befestigt ist. Rund um die Längsachse 20 und rund um die Bohrung 6 verläuft eine Wand 7, In der eine ringförmige Sacklochbohrung 8 vorgesehen ist.

Jener Teil der Wand 7, der sich vom Boden 10 der Sacklochbohrung 8 bis zum ersten Ende 4 des Grundkörpers 3 erstreckt, bildet ein Lichtabgabefenster 9. Das Lichtabgahefenster 9 Ist somit ein einstückig ausgeführter, integraler Teil des Grundkörpers 3, der aus einem Material besteht, das transparent für das von den LEDs 2 abgestrahlte oder konvertierte Licht Ist Das Lichtabgabefenster 9 endet am ersten Ende 4 in einer Lichtabgabefläche 18, die in den Figuren 1 - 3 als plane Fläche ausgebildet ist, auf der gegebenenfalls ein optisches Element, zum Beispiel eine Sammellinse anbringbar ist. Alternativ kann die Lichtabgabefläche 18 selbst als optisches Element dienen, d.h. zum Beispiel als Sammellinse geformt sein. Der Grundkörper 3 besteht aus Kunststoff, insbesondere aus Polyphenylsulfon, Polyethersulfon, Polycarbonat oder Polyamid oder aus Glas. Der Grundkörper 3 der Figur 1 hat in etwa eine Länge von 5 - 10 mm, einen Außendurchmesser am ersten Ende 4 von in etwa 11 mm und einen innendurchmesser der Bohrung 6 am ersten Ende 4 von in etwa 5 mm.

Auf dem ringförmigen Träger 19 sind vier optische Halbleiterbauelemente 2 in Form von Leuchtdioden (LEDs) In einem regelmäßigen Abstand von etwa 90° befestigt. Es können dabei Identische LEDs verwendet werden oder LEDs, die unterschiedliche Wellenlängen abstrahlen, so dass insgesamt ein im Wesentlichen weißes Licht emittiert wird. Der Träger 19 wird von einer ersten zentrischen Bohrung 21 und zwei weiteren, kleineren, exzentrischen Bohrungen 22 durchsetzt. Wie insbesondere aus den Figuren 2 und ersichtlich Ist, Ist der Außendurchmesser des Trägers 19, der Durchmesser der Bohrung 21 und somit die Wandstärke der ringförmigen Trägerwand derart bemessen, dass der Träger 19 mit den LEDs 2 in die Sacklochbohrung 8 einsetzbar ist. Durch die Bohrungen 22 verläuft jeweils ein elektrischer Kontakt als Teil der elektrischen Versorgungseinrichtung zur Stromversorgung der LEDs 2, die in Serie geschalten sind.

Abdeckvorrichtung 11 ist als ringförmiger Verschlusskörper 12 mit einer zentralen Bohrung 15, die vom zweiten Ende 14 zum ersten Ende 13 reicht, ausgebildet. Durch Bohrung 15 ragt, genauso wie durch Bohrung 21 des Trägers 19 die Werkzeugaufnahme 33, wenn die Beleuchtungsvorrichtung 1 am Handgriff 30 befestigt ist. In die ringförmigen Wand 16 der Abdeckvorrichtung 11 sind zwei kleine Bohrungen 17 zur Aufnahme der elektrischen Kontakte der elektrischen Versorgungseinrichtung eingebracht. In montiertem Zustand schließen die Bohrungen 17 an die Bohrungen 22 des Trägers 19 an. Die Abdeckvorrichtung 11 ist aus Kunststoff, insbesondere aus Polyphenylsulfon, Polyethersulfon, Polycarbonat oder Polyamid, Glas oder Keramik hergestellt ist. Die Abdeckvorrichtung 11 der Figur 2 hat in etwa eine Länge von 1 - 5 mm.

Wie aus den Figuren 2 und 3 erkennbar ist, ist ein Teil der Abdeckvorrichtung 11 in der Sacklochbohrung 8 des Grundkörpers 3 aufgenommen. Um das Einbringen der Abdeckvorrichtung in die Sacklochbohrung 8 zu erleichtern, weist das erste Ende 13 an seinem Außenumfang und am Umfang der Bohrung 15 Fasen auf. Durch eine dauerhaft dichte Verbindung der Abdeckvorrichtung 11 mit dem Grundkörper 3 entsteht aus der Sacklochbohrung 8 ein hermetisch abgedichteter Innenraum, in dem die LEDs 2 angeordnet sind. Die Verbindung kann durch Verpressen, Verkleben oder mittels Dichtelementen, zum Beispiel O-Ringen oder Gummiringen erfolgen. Bevorzugt erfolgt die Verbindung durch Verschweißen, besonders bevorzugt durch Laserverschweißen. Bei der Abdichtung mittels Dichtelementen können zur Aufnahme der Dichtelemente Ringnuten in der Wand 16 der Abdeckvorrichtung 11 und / oder in den Wänden der Sacklochbohrung 8 vorgesehen sein.

Bei Betrieb emittieren die LEDs 2 Strahlung in den hermetisch abgedichteten Innenraum, wobei die Strahlung über das transparente Lichtabgabefenster 9 und die Lichtabgabeoberfläche 18 an die Umgebung abgestrahlt wird. Im hermetisch abgedichteten Innenraum sind Funktionselemente zur Verbesserung der Lichtabgabe enthalten, die Lumineszenzfarbstoffe enthaltende Konvertierungspasten zur Umwandlung der von den LEDs 2 abgestrahlten Wellenlänge umfassen. In einem Beispiel umfassen die Funktionselemente zur Verbesserung der Lichtabgabe Filter oder Reflektoren zur Lenkung der Strahlung in Richtung des Lichtabgabefensters 9. Auch diese Funktionselemente sind somit vor Feuchtigkeit, Chemikalien und anderen schädlichen Umgebungseinflüssen geschützt.

Die Beleuchtungsvorrichtung 1' der Figur 3 unterscheidet sich von der Beleuchtungsvorrichtung 1 durch ihre deutlich geringere Bauhöhe, die durch geringer Längenabmessungen - bezogen auf die Längsachse 20 - des Grundkörpers 3' und der Abdeckvorrichtung 11' erzielt wird. Entsprechend ist auch das Lichtabgabefenster 9' schmäler ausgebildet. Um dennoch eine zuverlässige Kapselung des die Sacklochbohrung 8 umfassenden, hermetisch abgedichteten Innenraums zu erhalten, besteht die Wand 16 der Abdeckvorrichtung 11' aus einem ersten Abschnitt 16A mit einem geringeren Durchmesser und einem zweiten, flanschartigen Abschnitt 16B mit einem größeren Durchmesser. Die durch den flanschartigen Abschnitt 16B gebildeten Ringschultern 23 und 24 dienen als Auflager für Grundkörper 3' und insbesondere als eine hervorragende Verbindungsstelle zum Verschweißen des Grundkörpers 3' mit der Abdeckvorrichtung 11'.

Die Erfindung ist nicht auf die beschriebenen Ausführungsbeispiele beschränkt. Es wird auch ausdrücklich noch einmal darauf verwiesen, dass die Beleuchtungsvorrichtung nicht wie in den Figuren 1 - 3 dargestellt zwingend als Ringlicht, das ein Behandlungswerkzeug, eine Werkzeugaufnahme oder ein anderes Bauteil des Handgriffs umgibt und mit den Bohrungen 6, 15, 21 versehen ist, ausgebildet sein muss. Selbstverständlich kann eine Beleuchtungsvorrichtung gemäß der Erfindung auch ohne diese Bohrungen 6, 15, 21 hergestellt werden und beispielsweise an die Lichtabgabefläche 18 ein Lichtleiter angeschlossen werden, der das Licht an das werkzeugseitige Ende oder an eine andere gewünschte Lichtabgabestelle des Handgriffs weiterleitet.

## Patentansprüche

1. Medizinischer Handgriff (30), zum Beispiel ein gebogener Handgriff, ein Handgriff mit gewinkelt zueinander angeordneten Abschnitten, ein Motor, eine Kupplung, ein Adapter oder ein Zwischenstück, mit einer Beleuchtungsvorrichtung (1, 1') mit zumindest einem optischen Halbleiterbauelement (2) als Lichtquelle, wobei die Beleuchtungsvorrichtung (1, 1') des Weiteren die folgenden Teile umfasst:
- einen Grundkörper (3, 3') mit einem ersten Ende (4) und einem zweiten Ende (5),
- zumindest eine in dem Grundkörper (3, 3') angeordnete Sacklochbohrung (8), die sich vom zweiten Ende (5) des Grundkörpers (3, 3') in Richtung des ersten Endes (4) erstreckt,
- ein Lichtabgabefenster (9, 9'), das durch jenen Teil des Grundkörpers (3, 3'), der sich vom Boden (10) der Sacklochbohrung (8) bis zum ersten Ende (4) des Grundkörpers (3, 3') erstreckt, gebildet ist,
- eine Abdeckvorrichtung (11, 11'), die mit dem Grundkörper (3, 3') verbunden ist und mit der Sacklochbohrung (8) einen hermetisch abgedichteten Innenraum bildet,
wobei das zumindest eine optische Halbleiterbauelement (2) in dem hermetisch abgedichteten Innenraum angeordnet ist und über eine elektrische Versorgungseinrichtung mit einer Stromquelle verbunden oder verbindbar ist, wobei der Grundkörper (3, 3') und die Abdeckvorrichtung (11, 11') aus elektrisch nicht leitendem Material bestehen, **dadurch gekennzeichnet, dass** in dem hermetisch abgedichteten Innenraum Funktionselemente zur Verbesserung der Lichtabgabe enthalten sind, die Lumineszenzfarbstoffe enthaltende Konvertierungspasten zur Umwandlung der von dem zumindest einen optischen Halbleiterbauelement (2) abgestrahlten Wellenlänge umfassen.

2. Medizinischer Handgriff (30) nach Anspruch 1, **dadurch gekennzeichnet, dass** zumindest ein Teil der Abdeckvorrichtung (11, 11') in der zumindest einen Sacklochbohrung (8) des Grundkörpers (3, 3') aufgenommen ist.

3. Medizinischer Handgriff (30) nach Anspruch 1 oder 2, **dadurch gekennzeichnet, dass** die Abdeckvorrichtung (11, 11') einen Verschlusskörper (12) mit einem ersten Ende (13) und einem zweiten Ende (14) umfasst, wobei der Verschlusskörper (12) bevorzugt mit einer vom ersten Ende (13) zum zweiten Ende (14) reichenden Bohrung (15) und einer die Bohrung (15) umschließenden Wand (16) ausgebildet ist.

4. Medizinischer Handgriff (30) nach einem der vorhergehenden Ansprüche, **dadurch gekennzeichnet, dass**
der Grundkörper (3, 3') aus Kunststoff, insbesondere aus Polyphenylsulfon, Polyethersulfon, Polycarbonat oder Polyamid, oder Glas, die Abdeckvorrichtung (11, 11') aus Kunststoff, insbesondere aus Polyphenylsulfon, Polyethersulfon, Polycarbonat oder Polyamid, Glas oder Keramik hergestellt ist.

5. Medizinischer Handgriff (30) nach einem der vorhergehenden Ansprüche, **dadurch gekennzeichnet, dass**
der Grundkörper (3, 3') und die Abdeckvorrichtung (11, 11') miteinander verschweißt, verpresst, verklebt oder mittels Dichtelementen miteinander verbunden sind.

6. Medizinischer Handgriff (30) nach Anspruch 1 oder 2, **dadurch gekennzeichnet, dass** die Abdeckvorrichtung (11, 11') durch zumindest ein Vergussmaterial, insbesondere Kunstharze wie Epoxidharz, Silikonharze oder Polyamidharze gebildet ist.

7. Medizinischer Handgriff (30) nach einem der vorhergehenden Ansprüche, **dadurch gekennzeichnet, dass**
die elektrische Versorgungseinrichtung zumindest einen ersten und zweiten elektrischen Kontakt umfasst, die durch Bohrungen (17, 22) in der Beleuchtungsvorrichtung (1, 1') mit dem zumindest einen optischen Halbleiterbauelement (2) verbunden sind.

8. Medizinischer Handgriff (30) nach einem der Ansprüche 1 - 6, **gekennzeichnet durch** eine induktive elektrische Versorgungseinrichtung mit zumindest einer ersten Spule, die im hermetisch abgedichteten Innenraum der Beleuchtungsvorrichtung (1, 1') angeordnet ist, und zumindest einer zweiten Spule, die außerhalb des hermetisch abgedichteten Innenraums vorgesehen ist.

9. Medizinischer Handgriff (30) nach Anspruch 8, **dadurch gekennzeichnet, dass** zwischen der ersten Spule und dem zumindest einen optischen Halbleiterbauelement weitere elektronische Bauelemente, insbesondere Gleichrichter, Stromregler, AC-DC-Wandler, Kondensatoren oder Widerstände vorgesehen sind.

10. Medizinischer Handgriff (30) nach einem der vorhergehenden Ansprüche, **dadurch gekennzeichnet, dass**
die zumindest eine Sacklochbohrung (8) ausgebildet ist: als im wesentlichen zylindrischer Hohlraum, der nur unwesentlich größer als ein darin aufgenommenes optisches Halbleiterbauelement (2) ist; oder kreisbogenförmig und sich um die Längsachse (20) des Grundkörpers (3, 3') erstreckend; oder als ein durchgehender, rund um die Längsachse (20) des Grundkörpers (3, 3') verlaufender Ring.

11. Medizinischer Handgriff (30) nach Anspruch 6, **dadurch gekennzeichnet, dass** mehrere, vorzugsweise unterschiedliche optische Halbleiterbauelemente (2) in jeweils einer eigenen Sacklochbohrung (8) angeordnet und hermetisch verschlossen sind.

12. Medizinischer Handgriff (30) nach einem der vorhergehenden Ansprüche, **dadurch gekennzeichnet, dass**
die Beleuchtungsvorrichtung (1, 1') als Ringlicht ausgebildet ist, wobei zumindest der Grundkörper (3, 3') eine Bohrung (6) aufweist, in der das Werkzeug oder die Werkzeugaufnahme (33) aufnehmbar sind.

13. Medizinischer Handgriff (30) nach einem der vorhergehenden Ansprüche, **dadurch gekennzeichnet, dass**
das als Leuchtdiode ausgebildete zumindest eine optische Halbleiterbauelement (2) auf einer Platine (19) oder einer Multilayer-Keramik angeordnet ist.

14. Medizinischer Handgriff (30) nach einem der vorhergehenden Ansprüche, **dadurch gekennzeichnet, dass**
die Beleuchtungsvorrichtung (1, 1') im Inneren des Handgriffs angeordnet ist.

15. Verfahren zur Herstellung eines medizinischen Handgriffs (30), zum Beispiel eines gebogenen Handgriffs, eines Handgriffs mit gewinkelt zueinander angeordneten Abschnitten, eines Motors, einer Kupplung, eines Adapters oder eines Zwischenstücks, mit einer Beleuchtungsvorrichtung (1, 1'), mit den Schritten:
• Zur Verfügung stellen eines medizinischen Handgriffs (30),
• Zur Verfügung stellen einer Beleuchtungsvorrichtung (1, 1') mit zumindest einem optischen Halbleiterbauelement (2) als Lichtquelle, wobei die Beleuchtungsvorrichtung (1, 1') des Weiteren umfasst:
- einen Grundkörper (3, 3') mit einem ersten Ende (4) und einem zweiten Ende (5),
- zumindest eine in dem Grundkörper (3, 3') angeordneten Sacklochbohrung (8), die sich vom zweiten Ende (5) des Grundkörpers (3, 3') in Richtung des ersten Endes (4) erstreckt,
- ein Lichtabgabefenster (9, 9'), das durch jenen Teil des Grundkörpers (3, 3'), der sich vom Boden (10) der Sacklochbohrung (8) bis zum ersten Ende (4) des Grundkörpers (3, 3') erstreckt, gebildet ist,
- eine Abdeckvorrichtung (11, 11'), die mit dem Grundkörper (3, 3') verbunden ist und mit der Sacklochbohrung (8) einen hermetisch abgedichteten Innenraum bildet,
wobei das zumindest eine optische Halbleiterbauelement (2) im hermetisch abgedichteten Innenraum angeordnet ist und über eine elektrische Versorgungseinrichtung mit einer Stromquelle verbunden oder verbindbar ist, wobei der Grundkörper (3, 3') und die Abdeckvorrichtung (11, 11') aus elektrisch nicht leitendem Material bestehen,
• Befestigen der Beleuchtungsvorrichtung (1, 1') in oder an dem medizinischen Handgriff (30), **dadurch gekennzeichnet, dass**
in dem hermetisch abgedichteten Innenraum Funktionselemente zur Verbesserung der Lichtabgabe enthalten sind, die Lumineszenzfarbstoffe enthaltende Konvertierungspasten zur Umwandlung der von dem zumindest einen optischen Halbleiterbauelement (2) abgestrahlten Wellenlänge umfassen.

## Claims

1. A medical hand grip (30), such as a curved hand grip, a hand grip having sections that are arranged at an angle to one another, a motor, a coupling, an adapter or an intermediate piece, having an illumination device (1, 1') with at least one optical semiconductor component (2) serving as a light source, wherein the illumination device (1, 1') furthermore comprises the following components:
- a base body (3, 3') having a first end (4) and a second end (5),
- at least one blind hole (8) arranged in the base body (3, 3'), extending from the second end (5) of the base body (3, 3') in the direction of the first end (4),
- a light-emitting window (9, 9') formed by that part of the base body (3, 3') that extends from the bottom (10) of the blind hole (8) to the first end (4) of the base body (3, 3'),
- a cover device (11, 11') which is connected to the base body (3, 3') and together with the blind hole (8) forms a hermetically sealed interior,
wherein the at least one optical semiconductor component (2) is arranged in the hermetically sealed interior and is or can be connected to an electric power source via an electric power supply device, wherein the base body (3, 3') and the cover device (11, 11') are made of an electrically nonconducting material, **characterized in that** functional elements to improve the light emission are held in the hermetically sealed interior which comprise conversion pastes having luminescent pigments for converting the wavelength emitted by the at least one optical semiconductor component (2).

2. The medical hand grip (30) according to claim 1, **characterized in that** at least a portion of the cover device (11, 11') is accommodated in the at least one blind hole (8) of the base body (3, 3').

3. The medical hand grip (30) according to claim 1, **characterized in that** the cover device (11, 11') comprises a closure body (12) having a first end (13) and a second end (14), wherein the closure body (12) is preferably designed with a bore (15) extending from the first end (13) to the second end (14) and with a wall (16) surrounding the bore (15).

4. The medical hand grip (30) according to anyone of the preceding claims, **characterized in that**
the base body (3, 3') is made of plastic, in particular polyphenylsulfone, polyethersulfone, polycarbonate, or polyamide, or glass, the cover device (11, 11') is made of a plastic, in particular polyphenylsulfone, polyethersulfone, polycarbonate, or polyamide, or glass or ceramic.

5. The medical hand grip (30) according to anyone of the preceding claims, **characterized in that**
the base body (3, 3') and the cover device (11, 11') are welded, pressed or bonded together or are joined together by means of sealing elements.

6. The medical hand grip (30) according to claim 1 or 2, **characterized in that**
the cover device (11, 11') is formed by at least one casting compound, in particular synthetic resins such as epoxy resin, silicone resins or polyamide resins.

7. The medical hand grip (30) according to anyone of the preceding claims, **characterized in that**
the electric power supply device comprises at least one first and one second electric contact which are connected through bores (17, 22) in the illumination device (1, 1') to the at least one optical semiconductor component (2).

8. The medical hand grip (30) according to anyone of the claims 1 - 6, **characterized in that**
an inductive electric power supply device is provided with at least one first coil which is arranged in the hermetically sealed interior of the illumination device (1, 1') and with at least one second coil which is arranged outside of the hermetically sealed interior.

9. The medical hand grip (30) according to claim 8, **characterized in that** additional electronic components, in particular rectifiers, current regulators, AC-DC converters, capacitors or resistors, are provided between the first coil and the at least one optical semiconductor component (2).

10. The medical hand grip (30) according to anyone of the preceding claims, **characterized in that**
the at least one blind hole (8) is designed: as a substantially cylindrical hollow cavity that is only insignificantly larger than an optical semiconductor component (2) accommodated therein; having an arcuated shape and extending around the longitudinal axis (20) of the base body (3, 3'); or as a continuous ring running around the longitudinal axis (20) of the base body (3, 3').

11. The medical hand grip (30) according to claim 6, **characterized in that**
a plurality of, preferably different, optical semiconductor components (2) are arranged and hermetically sealed each in its own blind hole (8).

12. The medical hand grip (30) according to anyone of the preceding claims, **characterized in that**
the illumination device (1, 1') is designed as a ring light, wherein at least the base body (3, 3') comprises a bore (6) in which a tool or a tool receptacle (33) can be accommodated.

13. The medical hand grip (30) according to anyone of the preceding claims, **characterized in that**
the at least one optical semiconductor component (2) is designed as a LED which is arranged on a circuit board (19) or a multilayer ceramic.

14. The medical hand grip (30) according to anyone of the preceding claims, **characterized in that**
the illumination device (1, 1') is arranged in the interior of the hand grip.

15. A method for manufacturing a medical hand grip (30), such as a curved hand grip, a hand grip having sections that are arranged at an angle to one another, a motor, a coupling, an adapter or an intermediate piece, having an illumination device (1, 1'), comprising the steps:
• providing a medical hand grip (30),
• providing an illumination device (1, 1') having at least one optical semiconductor component (2) as a light source, wherein the illumination device (1, 1') additionally comprises:
- a base body (3, 3') having a first end (4) and a second end (5),
- at least one blind hole (8) arranged in the base body (3, 3') which extends from the second end (5) of the base body (3, 3') in the direction of the first end (4),
- a light-emitting window (9, 9') formed by that part of the base body (3, 3') which extends from a bottom (10) of the blind hole (8) to the first end (4) of the base body (3, 3'),
- a cover device (11, 11') which is connected to the base body (3, 3') and together with the blind hole (8) forms a hermetically sealed interior,
wherein the at least one optical semiconductor component (2) is arranged in the hermetically sealed interior and can be connected or is connected to an electric power source via an electric power supply device, wherein the base body (3, 3') and the cover device (11, 11') are made of an electrically nonconducting material,
• fastening the illumination device (1, 1') in or on the medical hand grip (30), **characterized in that**,
functional elements to improve the light emission are held in the hermetically sealed interior which comprise conversion pastes having luminescent pigments for converting the wavelength emitted by the at least one optical semiconductor component (2).

## Revendications

1. Manche médical (30), par exemple un manche recourbé, un manche avec des tronçons placés en étant coudés l'un par rapport à l'autre, un moteur, un accouplement, un adaptateur ou une pièce intermédiaire, avec un dispositif d'éclairage (1, 1') avec au moins un composant optique à semi-conducteur (2) en tant que source lumineuse, le dispositif d'éclairage (1, 1') comportant par ailleurs les éléments suivants:
- un corps de base (3, 3') avec une première extrémité (4) et une deuxième extrémité (5),
- au moins un perçage à trou borgne (8) placé dans le corps de base (3, 3') qui s'étend de la deuxième extrémité (5) du corps de base (3, 3') en direction de la première extrémité (4),
- une fenêtre d'émission lumineuse (9, 9') qui est formée par la partie du corps de base (3, 3') qui s'étend du fond (10) du perçage à trou borgne (8) jusqu'à la première extrémité (4) du corps de base (3, 3'),
- un dispositif de recouvrement (11, 11') qui est relié avec le corps de base (3, 3') et qui avec le perçage à trou borgne (8) forme un espace intérieur hermétiquement étanchéifié,
l'au moins un composant optique à semi-conducteur (2) étant placé dans l'espace intérieur hermétiquement étanchéifié et étant ou pouvant être relié par l'intermédiaire d'un système d'alimentation électrique avec une source électrique, le corps de base (3, 3') et le dispositif de recouvrement (11, 11') étant constitués d'une matière non conductrice d'électricité, **caractérisé en ce que** dans l'espace intérieur hermétiquement étanchéifié sont contenus des éléments fonctionnels pour l'amélioration de l'émission lumineuse qui comprennent de pâtes de conversion contenant des colorants luminescents pour la transformation de la longueur d'onde émise par l'au moins un composant optique à semi-conducteur (2).

2. Manche médical (30) selon la revendication 1, **caractérisé en ce qu'**au moins une partie du dispositif de recouvrement (11, 11') est réceptionnée dans l'au moins un perçage à trou borgne (8) du corps de base (3, 3').

3. Manche médical (30) selon la revendication 1 ou la revendication 2, **caractérisé en ce que**
le dispositif de recouvrement (11, 11') comprend un organe de fermeture (12) avec une première extrémité (13) et une deuxième extrémité (14), l'organe de fermeture (12) étant conçu de préférence avec un perçage (15) arrivant de la première extrémité (13) jusqu'à la deuxième extrémité (14) et une paroi (16) entourant le perçage (15).

4. Manche médical (30) selon l'une quelconque des revendications précédentes, **caractérisé en ce que**
le corps de base (3, 3') est fabriqué en matière plastique, notamment en polyphénylsulfone, en polyéthersulfone, en polycarbonate ou en polyamide ou en verre, le dispositif de recouvrement (11, 11') est fabriqué en matière plastique, notamment en polyphénylsulfone, en polyéthersulfone, en polycarbonate ou en polyamide, en verre ou en céramique.

5. Manche médical (30) selon l'une quelconque des revendications précédentes, **caractérisé en ce que**
le corps de base (3, 3') et le dispositif de recouvrement (11, 11') sont soudés, comprimés, collés l'un à l'autre ou reliés l'un à l'autre à l'aide d'éléments d'étanchéité.

6. Manche médical (30) selon la revendication 1 ou la revendication 2, **caractérisé en ce que**
le dispositif de recouvrement (11, 11') est formé par au moins une matière de scellement, notamment des résines synthétiques comme la résine époxy, des résines de silicone ou des résines de polyamide.

7. Manche médical (30) selon l'une quelconque des revendications précédentes, **caractérisé en ce que**
le système d'alimentation électrique comprend un premier et un deuxième contacts électriques, qui à travers des perçages (17, 22) dans le dispositif d'éclairage (1, 1') sont reliés avec l'au moins un composant optique à semi-conducteur (2).

8. Manche médical (30) selon l'une quelconque des revendications 1 à 6, **caractérisé par**
un système d'alimentation électrique inductif avec au moins une première bobine qui est placée dans l'espace intérieur hermétiquement étanchéifié du dispositif d'éclairage (1, 1') et au moins une deuxième bobine qui est prévue à l'extérieur de l'espace intérieur hermétiquement étanchéifié.

9. Manche médical (30) selon la revendication 8, **caractérisé en ce**
**qu'**entre la première bobine et l'au moins un composant optique à semi-conducteur sont prévus des composants électroniques supplémentaires, notamment des redresseurs, des rhéostats régulateurs d'intensité, des convertisseurs AC-DC, des condensateurs ou des résistances.

10. Manche médical (30) selon l'une quelconque des revendications précédentes, **caractérisé en ce que**
l'au moins un perçage à trou borgne (8) est conçu: comme une cavité sensiblement cylindrique qui n'est qu'a peine plus grande qu'un composant optique à semi-conducteur (2) qui y est réceptionné; ou en forme d'arc de cercle et s'étendant autour de l'axe longitudinal (20) du corps de base (3, 3'); ou en tant qu'un anneau s'étendant en continu en rond tout autour de l'axe longitudinal (20) du corps de base (3, 3').

11. Manche médical (30) selon la revendication 6, **caractérisé en ce que** plusieurs composants optiques à semi-conducteur (2) de préférence différents sont placés dans respectivement un propre perçage à trou borgne (8) et hermétiquement fermés.

12. Manche médical (30) selon l'une quelconque des revendications précédentes, **caractérisé en ce que**
le dispositif d'éclairage (1, 1') est conçu en tant qu'éclairage annulaire, au moins le corps de base (3, 3') comportant un perçage (6) dans lequel peuvent être réceptionnés l'outil ou le porte-outil (33).

13. Manche médical (30) selon l'une quelconque des revendications précédentes, **caractérisé en ce que**
l'au moins un composant optique à semi-conducteur (2) conçu en tant que diode électroluminescente est placé sur une carte (19) ou une céramique monocouche.

14. Manche médical (30) selon l'une quelconque des revendications précédentes, **caractérisé en ce que**
le dispositif d'éclairage (1, 1') est placé à l'intérieur du manche.

15. Procédé destiné à fabriquer un manche médical (30), par exemple un manche recourbé, un manche avec des tronçons placés en étant coudés l'un par rapport à l'autre, un moteur, un accouplement, un adaptateur ou une pièce intermédiaire, avec un dispositif d'éclairage (1, 1'), comportant les étapes:
• mise à disposition d'un manche médical (30),
• mise à disposition d'un dispositif d'éclairage (1, 1') avec au moins un composant optique à semi-conducteur (2) en tant que source lumineuse, le dispositif d'éclairage (1, 1') comportant par ailleurs :
- un corps de base (3, 3') avec une première extrémité (4) et une deuxième extrémité (5),
- au moins un perçage à trou borgne (8) placé dans le corps de base (3, 3') qui s'étend de la deuxième extrémité (5) du corps de base (3, 3') en direction de la première extrémité (4),
- une fenêtre d'émission lumineuse (9, 9') qui est formée par la partie du corps de base (3, 3') qui s'étend du fond (10) du perçage à trou borgne (8) jusqu'à la première extrémité (4) du corps de base (3, 3'),
- un dispositif de recouvrement (11, 11') qui est relié avec le corps de base (3, 3') et qui avec le perçage à trou borgne (8) forme un espace intérieur hermétiquement étanchéifié,
l'au moins un composant optique à semi-conducteur (2) étant placé dans l'espace intérieur hermétiquement étanchéifié et étant ou pouvant être relié par l'intermédiaire d'un système d'alimentation électrique avec une source électrique, le corps de base (3, 3') et le dispositif de recouvrement (11, 11') étant constitués d'une matière non conductrice d'électricité,
• fixation du dispositif d'éclairage (1, 1') dans ou sur le manche médical (30), **caractérisé en ce que**
dans l'espace intérieur hermétiquement étanchéifié sont contenus des éléments fonctionnels pour l'amélioration de l'émission lumineuse qui comprennent de pâtes de conversion contenant des colorants luminescents pour la transformation de la longueur d'onde émise par l'au moins un composant optique à semi-conducteur (2).
